(19) Europäisches Patentamt
European Patent Office
Office européen des brevets

(11) Veröffentlichungsnummer: **0 406 614 A1**

(12) **EUROPÄISCHE PATENTANMELDUNG**

(21) Anmeldenummer: **90111528.7**

(22) Anmeldetag: **19.06.90**

(51) Int. Cl.5: **C07D 409/12**, A61K 31/47, //(C07D409/12,333:00,215:00)

(30) Priorität: **05.07.89 AT 1639/89**

(43) Veröffentlichungstag der Anmeldung: **09.01.91 Patentblatt 91/02**

(84) Benannte Vertragsstaaten: **AT BE CH DE DK ES FR GB GR IT LI LU NL SE**

(71) Anmelder: **CHEMISCH PHARMAZEUTISCHE FORSCHUNGS-GESELLSCHAFT m.b.H.**
**St. Peter-Strasse 25**
**A-4021 Linz(AT)**

(72) Erfinder: **Binder, Dieter, Dr.**
**Sieveringerstrasse 207**
**A-1190 Wien(AT)**
Erfinder: **Rovenszky, Franz, Dipl.-Ing.Dr.**
**Lagerhausstrasse 5/8**
**A-2460 Bruck a.d. Leitha(AT)**
Erfinder: **Brunner, Norman, Dipl.-Ing.**
**Bergstrasse 66**
**A-2102 Hagenbrunn(AT)**
Erfinder: **Ferber, Hubert Peter, Dr.**
**Ahornweg 24**
**A-4021 Ansfelden(AT)**

(74) Vertreter: **Kunz, Ekkehard, Dr.**
**Chemie Holding AG Patentwesen St. Peter-Strasse 25**
**A-4021 Linz(AT)**

(54) **Neue Sulfamoyl-Thiophene, ein Verfahren zu ihrer Herstellung und ihre Verwendung.**

(57) Sulfamoyl-Thiophene der allgemeinen Formel

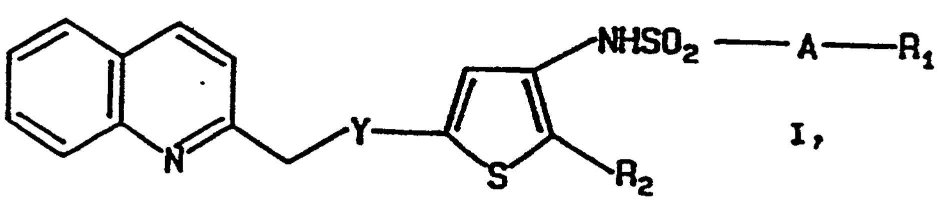

I,

in der
Y O oder S
A eine Einfachbindung oder eine geradkettige oder verzweigte Alkylengruppe mit 1 - 5 Kohlenstoffatomen,
$R_1$ Methyl oder Trifluormethyl und
$R_2$ Wasserstoff oder eine Gruppe COOH oder $COOR_3$ bedeutet, wobei
$R_3$ $(C_1-C_4)$-Alkyl darstellt,
und, für den Fall, daß $R_2$ eine Gruppe COOH bedeutet, ihre pharmazeutisch verträglichen Salze, ein Verfahren zur ihrer Herstellung, pharmazeutische Präparate, die diese Verbindungen enthalten sowie ihre Verwendung in

EP 0 406 614 A1

Medikamenten als Leukotrienantagonisten zur Behandlung von Asthma und Allergien.

## NEUE SULFAMOYL-THIOPHENE, EIN VERFAHREN ZU IHRER HERSTELLUNG UND IHRE VERWENDUNG

Die Erfindung betrifft neue Sulfamoyl-Thiophene, ein Verfahren zu ihrer Herstellung, pharmazeutische Präparate, die diese Verbindungen enhalten sowie ihre Verwendung in Medikamenten als Leukotrienantagonisten.

Gegenstand der Erfindung sind neue Sulfamoyl-Thiophene der allgemeinen Formel

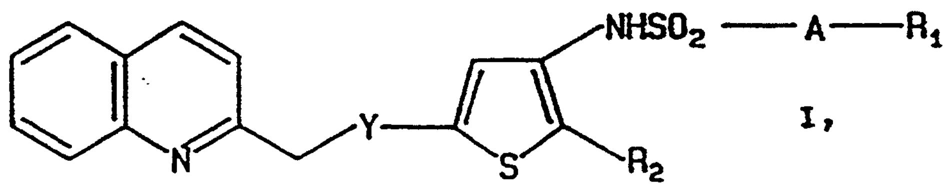

I,

in der
Y O oder S,
A eine Einfachbindung oder eine geradkettige oder verzweigte Alkylengruppe mit 1 - 5 Kohlenstoffatomen,
$R_1$ Methyl oder Trifluormethyl und
$R_2$ Wasserstoff oder eine Gruppe COOH oder $COOR_3$ bedeutetet, wobei
$R_3$ ($C_1$-$C_4$)-Alkyl darstellt,
und, für den Fall, daß $R_2$ eine Gruppe COOH bedeutet, ihre pharmazeutisch verträglichen Salze, ein Verfahren zu ihrer Herstellung, pharmazeutische Präparate, die diese Verbindungen enthalten sowie ihre Verwendung in Medikamenten als Leukotrienantagonisten.

Eine bevorzugte Klasse von Verbindungen der allgemeinen Formel I enthält jene Verbindungen, in denen A eine Einfachbindung.und $R_1$ Trifluormethyl bedeutet.

Besonders bevorzugte Einzelverbindungen sind:
5-(2-Chinolinylmethyloxy)-3-(1,1,1-trifluormethylsulfamoyl)-2-thiophencarbonsäuremethylester.
5-(2-Chinolinylmethyloxy)-3-(1,1,1-trifluormethylsulfamoyl)-2-thiophencarbonsäure.
1,1,1-Trifluor-N-[5-(2-chinolinylmethyloxy)-3-thienyl]methansulfonsäureamid.

Der in dieser Beschreibung verwendete Ausdruck ($C_1$-$C_4$)-Alkyl bezeichnet geradkettige oder verzweigte gesättigte Kohlenwasserstoffreste mit 1 bis 4 Kohlenstoffatmen, wie z.B Methyl, Ethyl, Propyl, Isopropyl, Butyl, Isobutyl, tert. Butyl.

Die Verbindungen der allgemeinen Formel I werden dadurch hergestellt, daß man

a) eine Verbindung der allgemeinen Formel

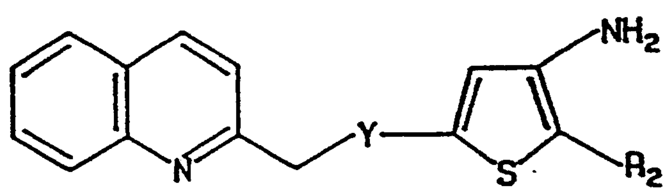

II,

in der Y O oder S und $R_2$ eine Gruppe $COOR_3$ bedeutet, wobei $R_3$ ($C_1$-$C_4$)-Alkyl darstellt, mit einer Verbindung der allgemeinen Formel

$X$-$SO_2$-A-$R_1$ III,

in der A und $R_1$ die obige Bedeutung haben und X Chlor oder eine Gruppe -O-$SO_2$-A-$R_1$ darstellt, wobei A und $R_1$ die obige Bedeutung haben, in einem reaktionsinerten organischen Lösungsmittel umsetzt,

b) erwünschtenfalls einen im Verfahrensschritt a) erhaltenen Ester der allgemeinen Formel I, in der Y, A und $R_1$ die obige Bedeutung haben und $R_2$ einen Rest $COOR_3$ darstellt, wobei $R_3$ ($C_1$-$C_4$)-Alkyl bedeutet, zu einer freien Säure der allgemeinen Formel I, in der $R_2$ eine Gruppe COOH bedeutet, verseift und gegebenenfalls mit anorganischen oder organischen Basen in ein pharmazeutisch verträgliches Salz überführt und

c) erwünschtenfalls eine im Verfahrensschritt b) erhaltene freie Säure der allgemeinen Formel I, in der $R_2$ eine Gruppe COOH darstellt und Y, A und $R_1$ die obige Bedeutung haben, zu einer Verbindung der Formel I, in der $R_2$ Wasserstoff bedeutet, decarboxyliert.

Die Umsetzung nach Verfahrensschritt a) wird am besten so durchgeführt, daß man eine Verbindung

der Formel II in einem reaktionsinerten, organischen Lösungsmittel, wie einem halogenierten organischen Lösungsmittel, z.B. Methylenchlorid oder Chloroform oder einem Ether, z.B. Diethylether, löst bzw. suspendiert, mindestens zwei Äquivalente einer anorganischen oder organischen Base wie Triethylamin, Pyridin, N-Methylmorpholin oder Trimethylsilanolat, bevorzugt Triethylamin oder Pydidin, zugibt und eine Lösung einer Verbindung der allgemeinen Formel III in gleichen Lösungsmittel bei einer Temperatur zwischen -80 und 30 °C, vorzugsweise zwischen -20 und 20 °C zutropft. Die Reaktionszeit liegt dann zwischen 30 Minuten und 4 Stunden, bevorzugt zwischen 30 - 90 Minuten.

Die so erhaltenen Ester der allgemeinen Formel I können auf übliche Weise mit alkoholischem, wäßrigen Alkali nach Verfahrensschritt b) zu den freien Carbonsäuren der Formel I verseift werden. Dazu löst man den Ester in einem Gemisch aus einem niederen aliphatischen Alkohol und Wasser und gibt zwei bis 6 Äquivalente Alkali, bevorzugt 2 bis 4 Äquivalente, zu. Man rührt dann bei einer Temperatur zwischen 30 und 100 °C. Die Reaktionszeit liegt dabei zwischen 2 und 24 Stunden, wobei den höheren Temperaturen die kürzeren Reaktionszeiten zuzuordnen sind.

Die erhaltenen Verbindungen der Formel I, in der $R_2$ eine Gruppe COOH bedeutet, können mit anorganischen oder organischen Basen in ihre pharmazeutisch verwendbaren Salze übergeführt werden. Die Salzbildung kann beispielsweise durchgeführt werden, indem man die genannten Verbindungen der Formel I in einem geeigneten Lösungsmitel, z.B. Wasser oder einem niederen aliphatischen Alkohol löst, eine äquivalente Menge der gewünschten Base zusetzt, für eine gute Durchmischung sorgt und nach beendeter Salzbildung das Lösungsmittel im Vakuum abdestilliert. Gegebenenfalls können die Salze nach der Isolierung umkristallisiert werden.

Pharmazeutisch verwendbare Salze sind z.B. Metallsalze, insbesondere Alkalimetall- oder Erdalkalimetallsalze, wie Natrium-, Kalium-, Magnesium - oder Calciumsalze. Andere pharmazeutisch verwendbare Salze sind beispielsweise auch leicht kristallisierende Ammoniumsalze. Letztere werden abgeleitet von Ammoniak oder organischen Aminen, z.B. Mono-, Di- oder Tri-nieder-(alkyl-, cycloalkyl- oder hydroxyalkyl-)aminen, Niederalkylendiaminen oder (Hydroxy-niederalkyl- oder Arylniederalkyl-)niederalkyl-Ammonium-Basen, z.B. Methylamin, Diethylamin, Triethylamin, Dicyclohexylamin, Triethanolamin, Ethylendiamin, Tris-(hydroxymethyl)-amino-methan, Benzyltrimethylammoniumhydroxid und dergleichen.

Die nach Verfahrensschritt b) erhaltenen freien Säuren oder deren Salze können zu Verbindungen der allgemeinen Formel I, in denen $R_2$ Wasserstoff bedeutet, decarboxyliert werden. Dazu wird das Ausgangsprodukt in einem geeigneten Lösungsmittel, wie in cyclischen oder aliphatischen Ethern oder in Pyridin, wäßriger Ammoniaklösung oder in einem niederen aliphatischen Alkohol gelöst oder suspendiert und zwischen 15 Minuten und 8 Stunden, bevorzugt zwischen 10 und 60 min, bei einer Temperatur zwischen 40 und 90 °C, bevorzugt zwischen 60 und 80 °C, unter guter Durchmischung erhitzt.

Die Aufarbeitung der erhaltenen Verbindungen erfolgt nach üblichen und jedem Fachmann geläufigen Methoden wie beispielsweise Extraktion, Fällung oder Umkristallisation.

Die Verbindungen der allgemeinen Formel II können gemäß dem folgenden Reaktionsschema und den speziellen Angaben in den Beispielen hergestellt werden.

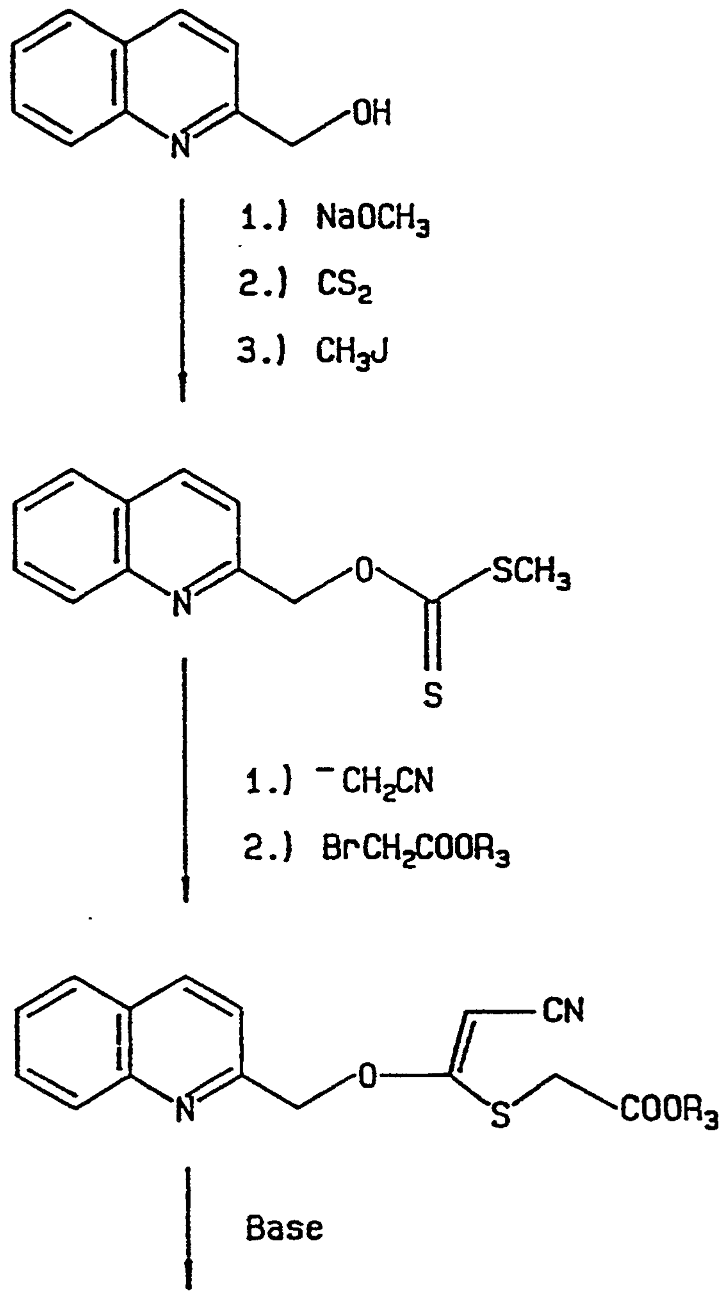


II

Die Sulfonsäurechloride oder -anhydride der allgemeinen Formel III sind literaturbekannt oder käuflich.

Die neuen Verbindungen der Formel I und, falls $R_2$ eine Gruppe COOH bedeutet, ihre pharmazeutisch verwendbaren Salze zeigen in in vivo und in vitro Modellen eine ausgezeichnete Hemmwirkung auf Leukotriene. Weiters hemmen sie die entzündlichen Prozesse bei chronischen Erkrankungen des Magen-Darm-Traktes, z.B. Morbus Crohn mit geringeren Nebenwirkungen als bekannte Leukotrienantagonisten.

Aufgrund dieser pharmakologischen Eigenschaften können die neuen Verbindungen allein oder in Mischung mit anderen Wirksubstanzen in Form üblicher galenischer Zubereitungen zur Behandlung von Krankheiten, die durch einen Überschuß an Leukotrienen verursacht werden, wie z.B. bei Asthma und Allergien, verwendet werden.

Die Verbindungen der Formel I sind zur Verwendung am Menschen bestimmt und können auf übliche Weise, wie beispielsweie oral, parenteral oder durch Inhalation verabreicht werden. Vorzugsweise werden sie durch Inhalation verabreicht, wobei die Tagesdosis ca. 10 mcg bis 10 mg/kg Körpergewicht beträgt, vorzugsweise 50 bis 500 mcg/kg Körpergewicht. Der behandelnde Arzt kann jedoch, abhängig vom

Allgemeinzustand und dem Alter des Patienten, der entspechenden Substanz der Formel I, der Art der Krankheit und der Art der Formulierung, auch Dosen darüber oder darunter verschreiben.

Falls die erfindungsgemäßen Substanzen zur Prophylaxe vewendet werden, bewegen sich die Dosen ungefähr im selben Rahmen wie im Behandlungsfall. Die Verabreichung durch Inhalation ist auch im Fall der Prophylaxe bevorzugt.

Die Verbindungen der Formel I können in Medikamenten allein oder in Verbindung mit anderen phamazeutisch aktiven Substanzen verabreicht werden, wobei der Gehalt der Verbindungen der Formel I zwischen 0,1 und 99 % liegt. Im allgemeinen liegen die pharmazeutisch aktiven Verbindungen in einer Mischung mit geeigneten inerten Hilfs- und/oder Trägerstoffen oder Verdünnungsmitteln, wie z.B. pharmazeutisch unbedenklichen Lösungsmitteln, Gelatine, Gummi arabicum, Milchzucker, Stärke, Magnesiumstearat, Talk, pflanzlichen Ölen, Polyalkylenglykol, Vaseline und dergleichen vor.

Die pharmazeutischen Präparate können in fester Form, beispielsweise als Tabletten, Dragees, Suppositorien, Kapseln und dergleichen, in flüssiger Form, beispielsweise als Lösungen, Suspensionen oder Emulsionen, in Zusammensetzungen mit verzögerter Freigabe des Wirkstoffes, oder in Formulierungen zur Inhalation, vorliegen. Gegebenenfalls sind sie sterilisiert und enthalten Hilfsstoffe, wie Konservierungs-, Stabilisierungs- oder Emulgiermittel, Salze zur Veränderung des osmotischen Druckes und dergleichen. Formulierungen in Spraydosen enthalten neben den oben genannten Stoffen Treibgase wie $CO_2$, Stickstoff oder halogenierte Kohlenwasserstoffe.

Insbesondere können pharmazeutische Präparate die erfindungsgemäßen Verbundungen in Kombination mit anderen therapeutisch wertvollen Stoffen enthalten. Mit diesen können die erfindungsgemäßen Verbindungen beispielsweise zusammen mit den oben angegebenen Hilfs- und/oder Trägerstoffen oder Verdünnungsmitteln zu Kombinationspräparaten formuliert werden.

**Beispiel 1:**

5-(2-Chinolinylmethyloxy)-3-(1,1,1-trifluormethylsulfamoyl)-2-thiophencarbonsäuremethylester

30,0 g (0,095 Mol) 3-Amino-5-(2-chinolinylmethyloxy)-thiophen-2-carbonsäuremethylester werden in 400 ml abs. Methylenchlorid bei Raumtemperatur suspendiert und unter Rühren mit 19,3 g (0,191 Mol) abs. Triethylamin versetzt. Die klare Lösung wird auf 0 °C abgekühlt. Innerhalb von 50 Minuten werden 47,1 g (0,167 Mol) in 50 ml abs. Methylenchlorid gelöstes Trifluorme thansulfonsäureanhydrid bei 0 °C zugetropft. Nach beendeter Zugabe wird 30 Minuten bei 0 °C gerührt und dann das Reaktionsgemisch auf Raumtemperatur erwärmen gelassen. Die braune Reaktionslösung wird mit 200 ml Methylenchlorid verdünnt und zweimal mit je 100 ml gesättigter Natriumbicarbonatlösung ausgeschüttelt. Die Methylenchlorid-phase wird zweimal mit je 100 ml 0,5n Salzsäure extrahiert und dann mit 50 ml gesättigter Natriumbicarbonatlösung ausgeschüttelt. Die vereinigten organischen Phasen werden über Natriumsulfat/Aktivkohle getrocknet, filtriert und eingedampft. Das Rohprodukt (40,5 g) wird aus 75 ml Ethanol kristallisiert.

Ausbeute: 23,3 g braune Kristalle (55 % d. Th., salzfrei)

Fp: 108 - 110 °C (Ethanol)

Das Ausgangsprodukt kann wie folgt hergestellt werden:

Dithiokohlensäure-O-(2-chinolinylmethyl)-S-methylester

80,0 g (0,503 Mol) 2-Chinolinmethanol [V. Bockelheide und W.J. Linn; J.Am.Chem.Soc. 76, 1286 (1954)] werden in 800 ml absolutem Methanol gelöst und mit 94 ml 5,4 m (0,506 Mol) Natriummethy-latlösung versetzt. Die klare Lösung wird eingedampft und im Feinvakuum getrocknet. Zum grauen, kristallinen Produkt (89,5 g) werden 600 ml Aceton zugegeben, die Suspension unter Rühren auf 0 °C abgekühlt und auf einmal mit 80,0 g (1,051 Mol).Schwefelkohlenstoff versetzt. Es wird noch 30 Minuten bei Raumtemperatur gerührt, wobei eine klare Lösung entsteht. Zu dieser Lösung werden 85,8 g (0,602 Mol) Methyljodid in einer Portion zugegeben. Es wird 20 Minuten bei 40 °C gerührt, dann wird die Reaktionslösung mit 15,0 g Natriumsulfit versetzt und eingedampft. Der Rückstand wird zwischen 700 ml gesättigter Natriumsulfitlösung und 500 ml Ether verteilt und die wäßrige Phase noch zweimal mit je .300 ml Ether extrahiert. Die vereinigten organischen Phasen werden einmal mit 400 ml Wasser gewachen, über Natriumsulfat/Altivkohle getrocknet und eingedampff. Das Rohprodukt (118,6 g) wird bei Raumtemperatur in 500 ml Diisopropylether gelöst, mit Aktivkohle versetzt, fil triert und bei -20 °C ausfallen gelassen. Das Produkt wird abgesaugt,

zweimal mit je 50 ml eiskaltem Diisopropylether digeriert und und drei Stunden bei 25 °C/1 mbar getrocknet.
Ausbeute: 103,2 g ockerfarbene Kristalle (82 % d. Th.)
Fp: 42 - 43 °C (Diisopropylether)

3-Amino-5-(2-chinolinylmethyloxy)-2-thiophencarbonsäuremethylester

231 ml (0,578 Mol) 2,5 m Butyllithium-Lösung in n-Hexan wird unter Rühren auf -80 °C abgekühlt und innerhalb von 25 Minuten mit 300 ml absolutem Tetrahydrofuran versetzt. Dann werden 22,2 g (0,539 Mol) trockenes Acetonitril, in 100 ml abs. Tetrahydrofuran gelöst, so zur BuLi.-Lösung zugetropft, daß die Temperatur nicht über -75 °C steigt (30 Minuten). Es wird 45 Minuten bei -80 °C nachgerührt. Dann werden 133,6 g (0,536 Mol) Dithiokohlensäure-O-(2-chinolinylmethyl)-S-methylester, gelöst in 300 ml absolutem Tetrahydrofuran, innerhalb von 35 Minuten zur Reaktionslösung so zugetropft, daß die Temperatur nicht über -80 °C steigt. Das Reaktionsgemisch wird eine Stunde bei -80 °C nachgerührt, dann auf 0 °C erwärmen gelassen und eingedampft. Das zurückbleibende orangerote Öl wird in 300 ml abs. Tetrahydrofuran gelöst, unter Rühren auf -30 °C abgekühlt und in einer Portion mit 84,9 g (0,555 Mol) Bromessigsäuremethylester, gelöst in 150 ml absolutem Tetrahydrofuran, versetzt, wobei die Temperatur auf 5 °C ansteigt. Das Reaktionsgemisch wird anschließend 150 Minuten unter Rückfluß erhitzt und dann eingedampft. Das ölige Rohprodukt wird zwischen 500 ml·gesättigter Natriumcarbonatlösung und 800 ml Methylenchlorid verteilt. Die Methylenchloridphase wird dreimal mit je 60 ml 0,5n Salzsäure extrahiert, über Natriumsulfat/Aktivkohle getrocknet und auf 0 °C abgekühlt. Unter Rühren wird das Endprodukt mit gasförmiger Salzsäure als Hydrochlorid gefällt. Es wird abgesaugt und zweimal mit je 100 ml kaltem Methylenchlorid gewaschen (75,4 g). Das Hydrochlorid wird in 500 ml Ethylacetat suspendiert und mit 500 ml Ethylacetat zweimal ex trahiert. Die vereinigten organischen Phasen werden über Natriumsulfat/Aktivkohle getrocknet, filtriert und eingedampft. Der Rückstand (57,2 g) wird aus 250 ml Ethanol, unter Verwendung von Aktivkohle, umkristallisiert.
Ausbeute: 48,6 g hellbraune Kristalle (29 % d.Th.)
Fp: 143 - 145 °C (Diisopropylether)

**Beispiel 2**

5-(2-Chinolinylmethyloxy)-3-(1,1,1-trifluormethylsulfamoyl)-2-thiophencarbonsäure

38,0 g (0,085 Mol) 5-(2-Chinolinylmethyloxy)-3-(1,1,1-trifluormethylsulfamoyl)-2-thiophencarbonsäuremethylester werden in 350 ml Methanol gelöst und mit ca. 100 ml 2n Natronlauge versetzt. Das Reaktionsgemisch wird 7 Stunden unter Rühren und Rückfluß erhitzt. Die Reaktionslösung wird auf 20 % ihres Volumens eingeengt und mit 500 ml Wasser verdünnt. Die wäßrige Phase wird viermal mit insgesamt 500 ml Ether extrahiert. Unter Rühren wird die wäßrige Phase auf 0 °C abgekühlt und mit 0,5n Salzsäure angesäuert. Das dabei ausgefallene Produkt wird abgesaugt, zweimal mit je 50 ml eiskaltem Ether digeriert und bei 60 °C/1 mbar getrocknet.
Ausbeute: 25,7 g hellbraune Kristalle (70 % d. Th.)
Fp: 101 - 102 °C (Zers.)

**Beispiel 3**

1,1,1-Trifluor-N-[5-(2-chinolinylmethyloxy)-3-thienyl]methansulfonsäureamid

22,0 g (0,051 Mol) 5-(2-Chinolinylmethyloxy)-3-(1,1,1-trifluormethylsulfamoyl)-thiophen-2-carbonsäure werden in 220 ml Wasser suspendiert und mit 22 ml conc. Ammoniak versetzt. Die klare Lösung wird mit Aktivkohle versetzt, filtriert, unter Rühren 15 Minuten auf 75 °C erhitzt und dann auf 35 °C abkühlen gelassen. Unter starkem Rühren wird mit 2n Salzsäure angesäuert und sofort mit 250 ml Ether überschichtet. Die wäßrige Phase wird noch dreimal mit je 200 ml Ether ausgerührt, die vereinigten Etherphasen werden über Natriumsulfat/Aktivkohlke getrocknet, filtriert und eingedampft. Das Rohprodukt (15,8 g) wird in

620 ml Diisopropylether bei Raumtemperatur gelöst, mit Aktivkohle versetzt, filtriert und bei -20 °C im Tiefkühlschrank auskristallisieren gelassen. Das Produkt wird abgesaugt und zweimal mit wenig eiskaltem Diisopropylether digeriert. Das Endprodukt wird bei 60 °C/1 mbar getrocknet.
Ausbeute: 10,3 g farblose Kristalle (52 % d. Th.)
Fp: 115,5 - 116,5 °C (Diisopropylether, Zers.)

**Beispiel 4**

Meerschweinchen wurden mit Urethan (1,4 g/kg i.p.) betäubt. Es wurde eine Kanüle in die Trachea eingebunden und mit einem Pneumotachographen (Fa. Fleisch) verbunden, der zur Messung des Atemflusses an einen Validyn Differenzdruck-Transducer (Modell DP 45-16) angeschlossen war. Der Intrapleuraldruck wurde kontinuierlich mit einem wassergefüllten Katheter gemessen, der in den Intrapleuralraum eingebunden und mit einem Validyn Druck-Transducer (Modell MPX -11 DP) verbunden war.

Die Daten wurden in einem Buxco Pulmonary Mechanics Analyzer (Modell 6) aufgezeichnet und ausgewertet, um Werte für Atemvolumen und Widerstand und Dehnbarkeit der Lunge zu erhalten.

Zur Verabreichung der Substanzen wurden Katheter in die V. jugularis und in das Duodenum eingebunden.

$LTD_4$ (Leukotrien $D_4$, 0,6 mcg/kg i.v.) wurde 10 Minuten nach der intravenösen oder 20 Minuten nach der intraduodenalen Gabe der Testsubstanzen verabreicht. Als Kontrolle dienten Tiere, die das Lösungsmittel ohne Substanzen erhielten. Alle Tiere wurden mit Indomethacin (10 mg/kg i.v.) und Propanolol (0,5 mg/kg i.v.) 20 bzw. 15 Minuten vor Verabreichung der Testsubstanzen vorbehandelt. Die Testsubstanzen wurden in einer Mischung von DMSO und 0,15 Mol/l $NaHCO_3$ (1:1) aufgelöst.

Pro Konzentrationswert wurden 4 Versuche durchgeführt und die Werte als Änderungen des Ausgangswertes in % angegeben. Die Ergebnisse sind in Tabelle 1 dargestellt.

Tabelle 1:

| | **Widerstand** | **Dehnbarkeit** |
|---|---|---|
| Kontrolle | + 1.018 % (± 525) | - 68 % (± 22) |
| 3 mg/kg i.v. | + 74 % (± 31) | - 23 % (± 7) |
| 5 mg/kg i.v. | + 27 % (± 9) | - 9 % (± 3) |
| 1 mg/kg i.d. | + 450 % (± 132,3) | - 43 % (± 17) |
| 3 mg/kg i.d. | + 172 % (± 96) | - 31 % (± 12) |
| 10 mg/kg i.d. | + 20 % (± 8) | - 18 % (± 9) |
| i.v.: intravenös<br>i.d.: intraduodenal | | |

**Ansprüche**

1. Sulfamoyl-Thiophene der allgemeinen Formel

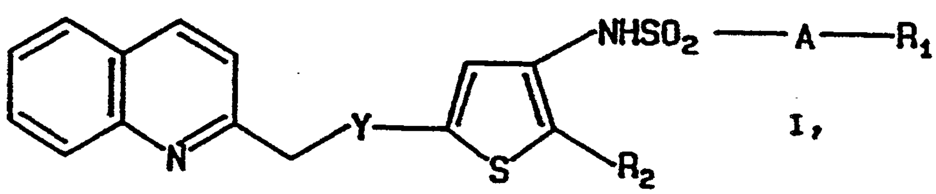

I,

in der
Y O oder S,
A eine Einfachbindung oder eine geradkettige oder verzweigte Alkylengruppe mit 1 - 5 Kohlenstoffatomen,

$R_1$ Methyl oder Trifluormethyl und
$R_2$ Wasserstoff oder eine Gruppe COOH oder $COOR_3$ bedeutet, wobei
$R_3$ $(C_1-C_4)$-Alkyl darstellt,
und, für den Fall, daß $R_2$ eine Gruppe COOH bedeutet, ihre pharmazeutisch verträglichen Salze.

2. Verbindungen der in Anspruch 1 definierten allgemeinen Formel I, in der A eine Einfachbindung und $R_1$ Trifluormethyl bedeutet.

3. 5-(2-Chinolinylmethyloxy)-3-(1,1,1-trifluormethylsulfamoyl)-2-thiophencarbonsäuremethylester.

4. 5-(2-Chinolinylmethyloxy)-3-(1,1,1-trifluormethylsulfamoyl)-2-thiophencarbonsäure.

5. 1,1,1-Trifluor-N-[5-(2-chinolinylmethyloxy)-3-thienyl]methansulfonsäureamid.

6. Verfahren zur Herstellung von Verbindungen der allgemeinen Formel I nach Anspruch 1, dadurch gekennzeichnet, daß man

a) eine Verbindung der allgemeinen Formel

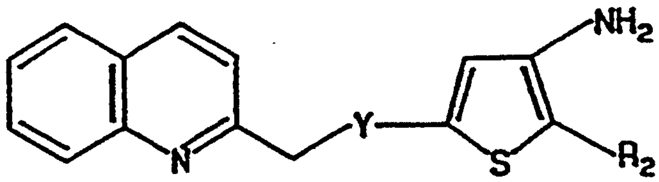

II,

in der Y O oder S und $R_2$ eine Gruppe $COOR_3$ bedeutet, wobei $R_3$ $(C_1-C_4)$-Alkyl darstellt, mit einer Verbindung der allgemeinen Formel

$X-SO_2-A-R_1$ III,

in der A und $R_1$ die obige Bedeutung haben und X Chlor oder eine Gruppe $-O-SO_2-A-R_1$ darstellt, wobei A und $R_1$ die obige Bedeutung haben, in einem reaktionsinerten organischen Lösungsmittel umsetzt,

b) erwünschtenfalls einen im Verfahrensschritt a) erhaltenen Ester der allgemeinen Formel I, in der Y, A und $R_1$ die obige Bedeutung haben und $R_2$ einen Rest $COOR_3$ darstellt, wobei $R_3$ $(C_1-C_4)$-Alkyl bedeutet, zu einer freien Säure der allgemeinen Formel I, in der $R_2$ eine Gruppe COOH bedeutet, verseift und gegebenenfalls mit anorganischen oder organischen Basen in ein pharmazeutisch verträgliches Salz überführt und

c) erwünschtenfalls eine im Verfahrensschritt b) erhaltene freie Säure der allgemeinen Formel I, in der $R_2$ eine Gruppe COOH darstellt und Y, A und $R_1$ die obige Bedeutung haben, zu einer Verbindung der Formel I, in der $R_2$ Wasserstoff bedeutet, decarboxyliert.

7. Pharmazeutische Präparate, enthaltend Verbindungen der allgemeinen Formel I nach Anspruch 1 oder, für den Fall, daß $R_2$ eine Gruppe COOH darstellt, deren pharmazeutisch verträgliche Salze, in Kombination mit üblichen galenischen Hilfs- und/oder Trägerstoffen oder Verdünnungsmitteln.

8. Pharmazeutische Präparate, enthaltend Verbindungen der allgemeinen Formel I nach Anspruch 1 oder, für den Fall, daß $R_2$ eine Gruppe COOH darstellt, deren pharmazeutisch verträgliche Salze, in Kombination mit anderen therapeutisch wertvollen Wirkstoffen sowie üblichen galenischen Hilfs-und/oder Trägerstoffen oder Verdünnungsmitteln.

9. Verbindungen der Formel I nach Anspruch 1 oder, für den Fall, daß $R_2$ eine Gruppe COOH darstellt, deren pharmazeutisch verträglichen Salze, zur Verwendung als Wirkstoffe für Arzneimittel zur Behandlung von Krankheiten, die durch Leukotrienantagonisten gelindert oder geheilt werden können.

Patentanspruch für folgende Vertragsstaaten: ES, GR

1. Verfahren zur Herstellung neuer Sulfamoyl-Thiophene der allgemeinen Formel

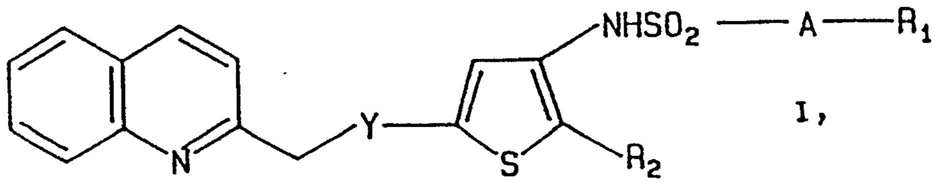

I,

in der
Y O oder S,

A eine Einfachbindung oder eine geradkettige oder verzweigte Alkylengruppe mit 1 - 5 Kohlenstoffatomen,
$R_1$ Methyl oder Trifluormethyl und
$R_2$ Wasserstoff oder eine Gruppe COOH oder $COOR_3$ bedeutet, wobei
$R_3$ ($C_1$-$C_4$)-Alkyl darstellt,
und, für den Fall, daß $R_2$ eine Gruppe COOH bedeutet, ihrer pharmazeutisch verträglichen Salze, dadurch gekennzeichnet, daß man

a) eine Verbindung der allgemeinen Formel

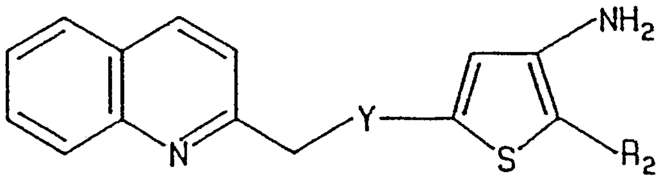

II,

in der Y O oder S und $R_2$ eine Gruppe $COOR_3$ bedeutet, wobei $R_3$ ($C_1$-$C_4$)-Alkyl darstellt, mit einer Verbindung der allgemeinen Formel

$X-SO_2-A-R_1$ III,

in der A und $R_1$ die obige Bedeutung haben und X Chlor oder eine Gruppe $-O-SO_2-A-R_1$ darstellt, wobei A und $R_1$ die obige Bedeutung haben, in einem reaktionsinerten organischen Lösungsmittel umsetzt,

b) erwünschtenfalls einen im Verfahrensschritt a) erhaltenen Ester der allgemeinen Formel I, in der Y, A und $R_1$ die obige Bedeutung haben und $R_2$ einen Rest $COOR_3$ darstellt, wobei $R_3$ ($C_1$-$C_4$)-Alkyl bedeutet, zu einer freien Säure der allgemeinen Formel I, in der $R_2$ eine Gruppe COOH bedeutet, verseift und gegebenenfalls mit anorganischen oder organischen Basen in ein pharmazeutisch verträgliches Salz überführt und

c) erwünschtenfalls eine im Verfahrensschritt b) erhaltene freie Säure der allgemeinen Formel I, in der $R_2$ eine Gruppe COOH darstellt und Y, A und $R_1$ die obige Bedeutung haben, zu einer Verbindung der Formel I, in der $R_2$ Wasserstoff bedeutet, decarboxyliert.

Europäisches Patentamt

# EUROPÄISCHER RECHERCHENBERICHT

Nummer der Anmeldung

**EP 90 11 1528**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 229 660 (CHEMIE LINZ AG)<br>* Ansprüche 1,8,9; Seite 5, Zeilen 3-12; Beispiele 1-7 * | 1,7,9 | C 07 D 409/12<br>A 61 K 31/47 //<br>(C 07 D 409/12<br>C 07 D 333:00<br>C 07 D 215:00 ) |
| A,P | CHEMICAL ABSTRACTS Band 112, Nr. 5, 29. Januar 1990, Seite 559, Zusammenfassung Nr. 35651w, Columbus, Ohio, US; J.H. MUSSER et al.: "N-((Arylmethoxy)phenyl) carboxylic acids, hydroxamic acids, tetrazoles, and sulfonyl carboxamides. Potent orally active leukotriene D4, antagonists of novel structure"<br>& J. Med. Chem. 1990, Band 33, Nr. 1, Seiten 240-245 | 1,9 | |
| A | PATENT ABSTRACTS OF JAPAN Bamd 12, Nr. 462 (C-549)(3309), 5. Dezember 1988;<br>& JP-A-63183563 (MECT CORP.) 28.07.1988 | 1 | |

| RECHERCHIERTE SACHGEBIETE (Int. Cl.5) |
|---|
| C 07 D 409/00 |

Der vorliegende Recherchenbericht wurde für alle Patentansprüche erstellt

| Recherchenort | Abschlußdatum der Recherche | Prüfer |
|---|---|---|
| Berlin | 15 Oktober 90 | HASS C V F |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument